# EUROPEAN PATENT SPECIFICATION

(11) **EP 3 087 951 B1**
(45) Date of publication and mention of the grant of the patent: **04.04.2018**
(21) Application number: 15166036.2
(22) Date of filing: 30.04.2015
(51) Int. Cl.: A61F 2/12, A61B 17/34

(54) **BREAST IMPLANT INTRODUCER**
BRUST-IMPLANTAT EINFÜHRVORRICHTUNG
INTRODUCTEUR D'IMPLANTS MAMMAIRES

(43) Date of publication of application: 02.11.2016
(73) Proprietor: Spronken, Caius Leonard Anthony, 400 Erts - La Massana (AD)
(72) Inventor: Spronken, Caius Leonard Anthony, 400 Erts - La Massana (AD)
(74) Representative: LC Patents

(56) References cited:
- EP-A2- 1 266 844
- DE-U1- 8 618 164
- FR-A1- 2 733 903
- GB-A- 2 454 325
- KR-A- 20110 013 656
- US-A- 5 615 807
- US-A1- 2002 091 443

## Description

### FIELD OF THE INVENTION

The present invention relates to an implant introducer, more in particular a breast implant introducer. Said implant introducer comprising a hollow tube and a tube holder, and being characterized in that it comprises attachment means and connecting means, wherein said connecting means allow said hollow tube to be partially disconnected from said tube holder, upon release of said attachment means.

### BACKGROUND TO THE INVENTION

Nowadays, breast augmentation surgery is becoming increasingly popular. The last five years the number of breast augmentations rose by as much as 36 percent in Europe. Due to the increasing demand for breast augmentations, intensive research is being performed to methods and devices allowing for the easy insertion of breast implants or breast prostheses.

Manual insertion of a breast implant has several disadvantages. For example a large incision is needed for manually introducing the implant into the body cavity. The larger the incision, the higher the risk of infections during and after surgery and the longer the recovery time. Furthermore, any (accidental) contact of the implant with a patient's skin provides a risk of connective tissue being dragged in the wound. Connective tissue can regrow into skin thereby forming cysts. Hence, one advantageous uses an implant for introducing a breast implant into the patient.

When developing such devices, several aspects should be kept in mind. For example, and in particular for the benefit to the patient, the incision area should be kept as small as possible to reduce the risk of post-surgery infections, and to avoid large visible scares. However, a small incision area increases the difficulty for the surgeon to introduce a rather large implant into the body cavity. Therefore most implant introducers comprise a hollow tube which have a rather large opening at one side for introducing the implant, and a smaller opening at the other side for pushing the implant through the opening, into a small incision in the patient.

For example DE8618164, US4955906, US5201779, FR2733903, WO2005023148, WO2005091831, and US7935089 all provide implant introducers having a hollow tube, for receiving an implant, and a piston, for driving the implant out of the device. Neither of these references provide an implant introducer comprising a tube holder with releasing means that allow the hollow tube to be fully or partially disconnected from said tube holder. The inventors have found that this novel system has particular advantages over the prior art known systems.

In particular, there are several types and sizes of breast implants available, which might all require a specifically adapted hollow tube and injection opening for optimal introduction into the body cavity, without damaging the implant. For example, a large breast implant will require a wider injection opening in comparison to a smaller breast implant in order to reduce the risk of overstretching or damaging the implant. Furthermore, different kind of implants have different stretching capabilities and require different injection openings. At all times, the smaller the injection opening, the better for the patient.

Due to the improvements under this invention, the tube holder, plunger, and driving mechanism can be kept standard for several different types and sizes of implants, while the hollow tube with its adapted injection opening, is easily interchangeable. As such the system of the present invention allows the tube holder and plunger to be made from a sustainable material such as stainless steel, while the hollow tube and injection opening may be made from a single-use material such as biocompatible plastics. In a specific embodiment, the single-use hollow tube could even be preloaded with the implant, thereby reducing the manual handling of the implant, and significantly increasing the sterility during operation.

Document US2002/0091443 describes an endoscopic prosthetic implant instrument for inserting a prosthetic breast implant, including a hollow sleeve member, a pusher member and a clam shell mechanism.
UK patent application GB 2 454 325 describes a device for delivering a biomedical implant comprising a barrel, a plunger and an inner sleeve.
Document FR 2 733 903 discloses an implant introducer according to the preamble of claim 1.

### SUMMARY OF THE INVENTION

In a first aspect, the present invention provides a breast implant introducer (1) according to claim 1.

In a further embodiment, said implant introducer further comprises a driving mechanism attached to said piston, for driving the displacement of said plunger within said hollow tube.

In a particular embodiment, said releasing means may comprise a first attachment member (13a) located at the first longitudinal end of said hollow tube, which is adapted to cooperate with a second attachment member (13b) located at the tube holder, which allow the hollow tube to be fully disconnected from the tube holder.

In a particular embodiment, said releasing means comprise attachment means (13) and connecting means (14), wherein said connecting means may allow the hollow tube to be partially disconnected from said tube holder, upon release of said attachment means.

In a particular embodiment, said attachment means comprises a first attachment member (13a) located at the first longitudinal end of said hollow tube, which is adapted to cooperate with a second attachment member (13b) located at the tube holder. More in particular said first and second attachment member comprise: a tab and an aperture adapted for receiving said tab.

In a further particular embodiment, said connecting means comprise a first connecting member (14a) located at the first longitudinal end of said hollow tube, which is adapted to cooperate with a second connecting member (14b) located at the tube holder. More in particular said first and second connecting members comprise: a hook and a pin adapted for receiving said hook; or a pin and an aperture adapted for receiving said pin.

In yet a further embodiment, said connecting means are located at the bottom part of the introducer. In another particular embodiment, said attachment means are located at the upper part of the introducer. Preferably, said attachment means and said connecting means are located substantially opposite with respect to each other.

In a further embodiment, said plunger is adapted to conform to the inner contours of the tapered flange (6) of said hollow tube.

Without being bound to particular measures or sizes, the different parts of the implant introducer are preferably provided in one or more of the following dimensions:
- the inner diameter of said hollow tube is about and between 60-100 mm, preferably about and between 70 - 90 mm, more preferably about 80 mm; and/or
- the diameter of said injection opening is about and between 10-50 mm, preferably about and between 20-40 mm, more preferably about 25 mm; and/or
- said injection opening ends into an injection tip being about and between 1-30 mm in length, preferably about 15 mm in length; and/or
- the hollow tube is between and about 10-50 cm in length, preferably about and between 10-40 cm, more preferably about 25 cm; and/or
- the angle of the tapered flange, represented by α, is about 30-60°, more preferably about 45; and/or
- the width of the push rod is between and about 5-20 mm, preferably about 10mm
- the cross section of the hollow tube is stadium-shaped, cylindrical, oval, concave or convex in shape; preferably stadium-shaped or cylindrical; and/or
- the cross section of the injection tip is stadium-shaped.

Due to its use during surgical procedures, the implant introducer of the present invention is preferably made from a sterilisable biocompatible material such as for example selected from the non-limiting list comprising stainless steel, poly-urethane, poly-propylene poly-ethylene, glass or acrylics.

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.

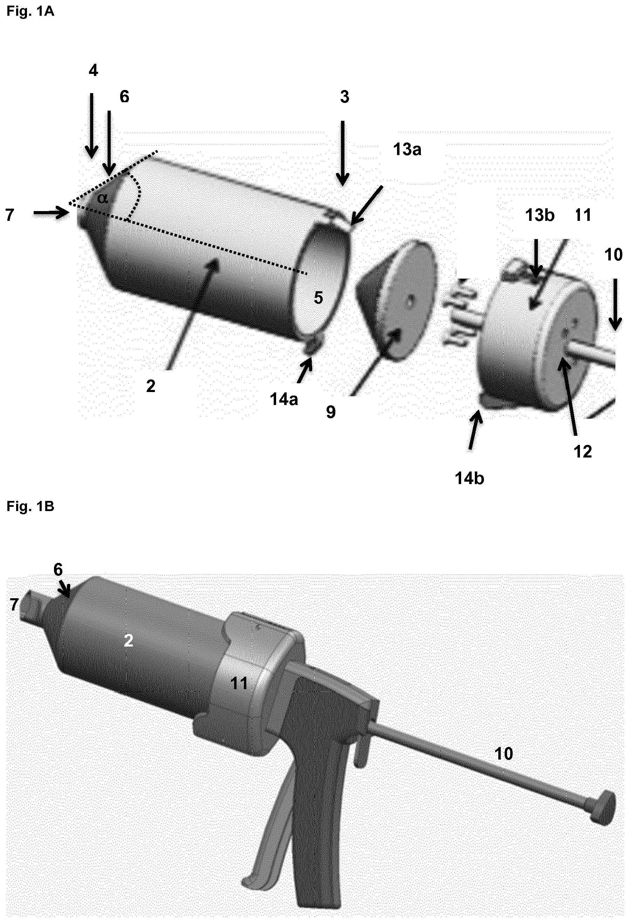

**Fig. 1****:** Represents a first (A) and second (B) embodiment of the implant introducer according to the present invention.
**Fig. 2****:** Represents a first embodiment of a driving mechanism suitable for driving the piston of the implant introducer of the present invention.
**Fig. 3****:** Represents a first embodiment of the first (13a) and second (13b) attachment members suitable in the context of the present invention.
**Fig. 4****:** Represents a first (A) and second (B) embodiment of the first (14a) and second (14b) connecting members suitable in the context of the present invention.
**Fig. 5****:** Represents a schematic indication of the angle α, in the context of the present invention.
**Fig. 6****:** Represents a first (A) and second (B) embodiment of the shape of the injection opening (7) of the injection tip according to the present invention.
**Fig. 7****:** Represents a first (A) and second (B) embodiment of the shape of the transitions, i.e.
sharp (A) or smooth (B), between the hollow tube (2), the tapered flange (6) and the injection tip (7).
**Fig. 8****:** Represents a first (A) and second (B) embodiment of the shape of the injection tip, i.e.
having equally rectangular sides (A) or rectangular sides which are different in height/length (B), such as the shape of a needle, but without sharp edges.
**Fig. 9****:** Represents a first (A), second (B) and third (C) embodiment of the shape of the plunger (9) in the context of the present invention.
**Fig. 10****:** Represents an embodiment of the attachment means in the context of the invention, i.e. including an elevated ring (21) and a spring-actuated pivoting member (22).

### Numbering used with reference to the specific parts of the device:

| **No** | **Part** | **N°** | **Part** |
|---|---|---|---|
| (1) | Implant introducer | (14) | Connecting means |
| (2) | Hollow tube | (14a) | First connecting member |
| (3) | First longitudinal end of hollow tube | (14b) | Second connecting member |
| (4) | Second longitudinal end of hollow tube | (15a) | First scum |
| (5) | Receiving opening | (15b) | Second scum |
| (6) | Tapered flange | (16a) | First spring |
| (7) | Injection opening | (16b) | Second spring |
| (8) | Piston | (17) | Slide |
| (9) | Plunger | (18) | Pivot point |
| (10) | Pushrod | (19) | Pad |
| (11) | Tube holder | (20) | Pinching arm |
| (12) | Opening for receiving pushrod | (21) | Elevated ring |
| (13) | Attachment means | (22) | Spring-actuated pivoting member |
| (13a) | First attachment member | (23) | Tapered flange |
| (13b) | Second attachment member | (24) | Groove |

### DETAILED DESCRIPTION OF THE INVENTION

As already indicated herein before, the present invention in a first aspect provides an implant introducer (1) comprising:
- a hollow tube (2) comprising a first (3) and second longitudinal end (4), said first longitudinal end having a receiving opening (5) for receiving said implant; said second longitudinal end having a tapered flange (6) and an injection opening (7) for injecting said implant;
- a piston (8) comprising a plunger (9) and a pushrod (10); said plunger being adapted to conform to the inner contours of said hollow tube;
- a tube holder (11) having an opening (12) for receiving the pushrod of said piston;
characterized in that said implant introducer further comprises releasing means (13, 14), which allow the hollow tube to be fully or partially disconnected from said tube holder.

As discussed herein before, the improvements of the device according to this invention reside in the presence of a tube holder having releasing means, which allow the hollow tube to be fully, or partially disconnected from said tube holder. The prior art devices do not have these features, since most of these are very simple devices, very much looking like an enlarged syringe. The surgeon will then have to push out the implant by actuating the plunger with his thumb, which is often very hard due to the fact that a rather large implant needs to be squeezed through a small injection opening into a small incision in the patient. Furthermore, preferably for each type and size of implant a suitably adapted hollow tube and injection opening is provided, since large implants will require a larger injection opening in comparison to smaller implant. Hence, the simpler the device, the lower the costs for providing several different types. Nevertheless, one remains faced with the difficulty of squeezing out the implant manually.

Due to the improvements under this invention, the tube holder, piston and driving mechanism can be kept standard for several different types and sizes of implants, while the hollow tube with its adapted injection opening, is easily interchangeable. As such a more complex and expensive driving mechanism can be provided, which solves the problem of squeezing out the implant manually, while at the same time a high degree of flexibility is provided for adapting the device according to the explicitly chosen implant type and size, by means of the interchangeable hollow tubes.

As such the system of the present invention would allow the tube holder, plunger, and driving mechanism to be made from a sustainable material such as stainless steel, while the hollow tube and injection opening may be made from a single-use material such as biocompatible plastic. In a specific embodiment, the single-use hollow tube could then even be preloaded with the implant, thereby reducing the manual handling of the implant, and significantly increasing the sterility during operation.

The implant introducer is suitable for introducing a variety of implants of varying materials, such as for example silicon implants, hydrogel implants, saline implants, polyurethane implants,.... In a particular embodiment, said implant is a breast implant, more in particular a silicone breast implant.

As discussed herein above, during surgery, it is essential to work not only quick, but also as sterilize as possible to avoid contamination of the device and post-surgery infections in a patient. Therefore, it could be advantageous to provide a device, in which the hollow tube can be partially disconnected from the tube holder during its use. As such introducing of the implant in the device can occur in a fast and sterile fashion, without any parts of the device being placed aside with a risk of contamination. Therefore, in a particular embodiment, said releasing means comprise attachment means (13) and connecting means (14), wherein said connecting means allow the hollow tube to be partially disconnected from said tube holder, upon release of said attachment means.

To this extend the device is provided with attachment means by which the hollow tube can be attached or detached (released) from the tube holder, and optionally further comprises connecting means which allow the hollow tube to remain connected with the tube holder, when releasing the attachment means.

In a particular embodiment, said attachment means comprises a first attachment member (13a) located at the first longitudinal end of said hollow tube, which is adapted to cooperate with a second attachment member (13b) located at the tube holder.

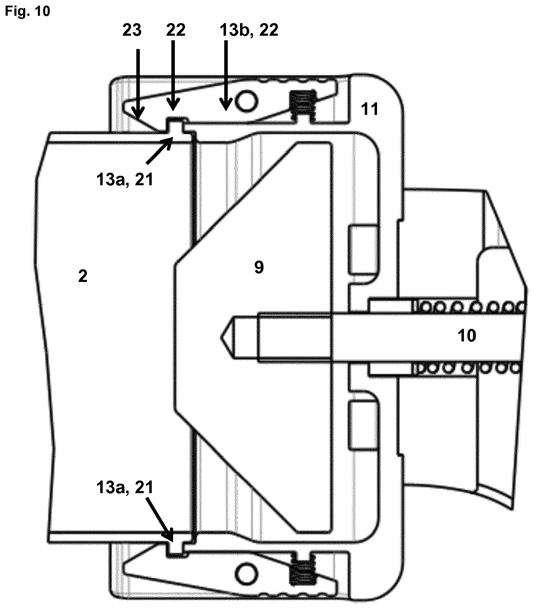

In a first particular embodiment, said attachment means may comprise a first attachment member (13a) which consists of an elevated ring (21) at the outer surface of the hollow tube near the opening for receiving the pushrod (12) (see figure 10). In said embodiment, the second attachment member (13b), which is located at the inner surface of the tube holder, consists of a spring-actuated pivoting member (22) having a tapered flange (23) and a groove (24), said groove adapted to confirm to the size and shape of the elevated ring (21). By inserting the hollow tube in the tube holder, the elevated ring (21) will slide along the tapered flange (23) of the spring-actuated pivoting member (22), until it reaches the end of the tapered flange (23) and is locked into the groove (24). For releasing the hollow tube, the spring-actuated pivoting member can be pushed down thereby releasing the elevated ring (21) from the groove (24). Although the elevated ring (21) is preferably continuous, it may be interrupted, such that it consists of several parts. The advantage of a continuous ring, is that it allows to rotate the hollow tube once it is in place to adapt the device to the preferred angle of the surgeon. Furthermore, although the tube holder may comprise one or more, in particular 1, 2, 3, or 4 spring-actuated pivoting members, it preferably comprises 2 opposite spring-actuated pivoting members, for easy access by the person handling the device.

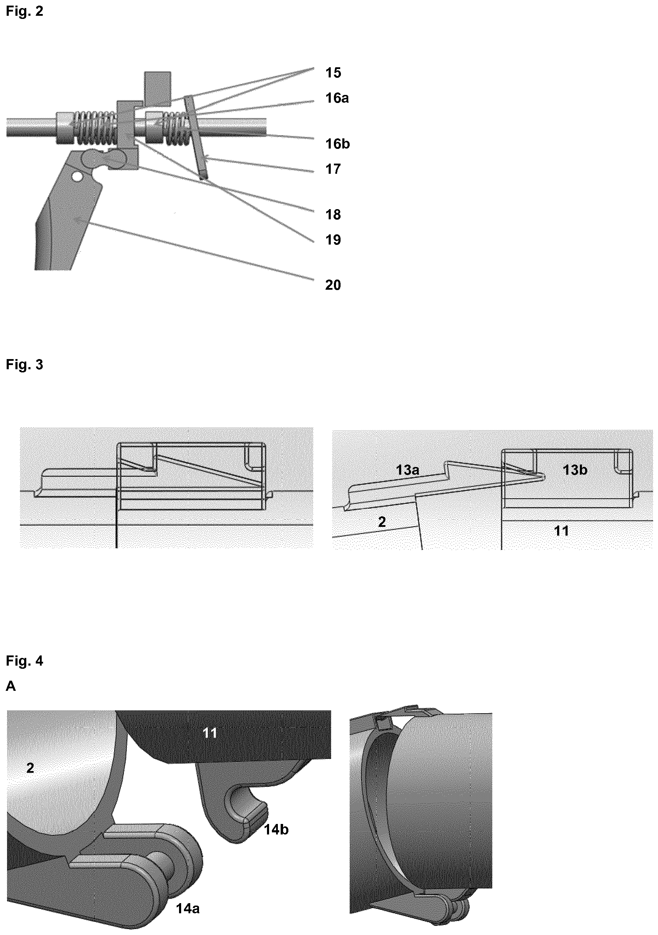

In a second particular embodiment, said first and second attachment member may comprise: a tab and an aperture adapted for receiving said tab, such as for example depicted in figure 3. In this embodiment, the hollow tube can be partially released from the housing by pushing down the tab, while at the same time pushing back the hollow tube. In contrast, the hollow tube can again be fully attached to the tube holder when the tab cooperates with the aperture, and thus cooperates with the housing of the tube holder. This process of attachment and/or detachment of the hollow tube from the tube holder is preferably done when the piston is in its most retracted position, i.e. the plunger being located as closely as possible to the tube holder.

The attachment mechanism comprising a tab and an aperture should be designed as such that the tab is firmly fit into the aperture, while at the same time being slightly bendable to the extend that it does not break off, but is sufficiently flexible to allow the tab to be pushed out of the aperture, without too much force needed by the user. Other types of attachment means can of course also be provided, in as far as they allow the hollow tube and tube holder to be attached and partially detached from each other with a simple movement by the user, while they should prevent the accidental, non-intended, detachment during use. Such alternative attachment means may include screws, clips, bayonets,...

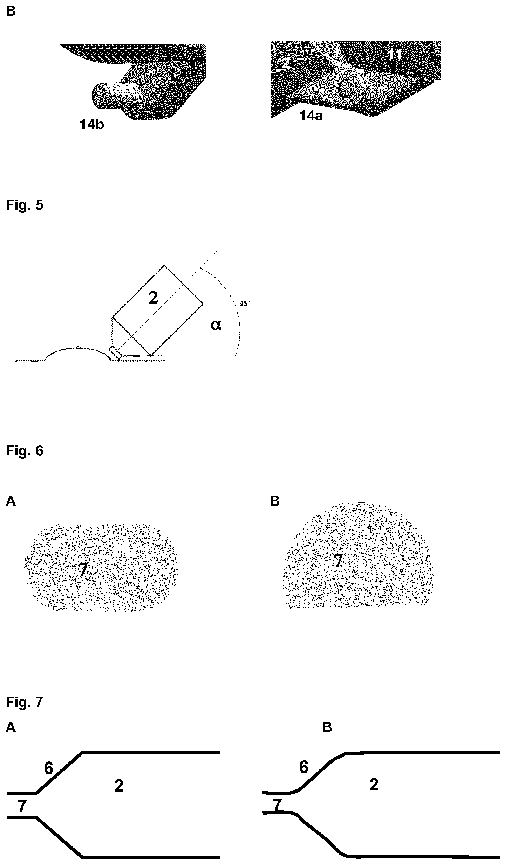

In a further embodiment, the connecting means comprise a first connecting member (14a) located at the first longitudinal end of said hollow tube, which is adapted to cooperate with a second connecting member (14b) located at the tube holder. More in particular said first and second connecting members may comprise: a hook and a pin adapted for receiving said hook (see for example figure 4A); or a pin and an aperture adapted for receiving said pin (see for example figure 4B).

In the embodiment as depicted in figure 4A, a hook is provided which grasps around a pin, thereby providing a hinge mechanism around which the hollow tube is allowed to pivot, but at the same time remains connected to the hollow tube. This embodiment has the advantage that the pin is attached to the device at both ends, such that the forces applied to the pin are evenly distributed. In the embodiment as depicted in figure 4B, a pin is provided which fits into an aperture, thereby providing a hinge mechanism around which the hollow tube is allowed to pivot, but at the same time remains connected to the hollow tube. Both embodiments allow the user to easily insert the implant into the device, without the need of temporarily placing parts aside for further use.

Other types of connecting means can of course also be provided, in as far as they allow the hollow tube and tube holder to remain connected with each other after detaching the hollow tube and tube holder using the attachment means.

Although the attachment (13) and connecting (14) means can be located at varying positions in the device, the connecting means are preferably located at the bottom part of the implant introducer, wherein the bottom part is referred to as the underside of the device when held in a position suitable for use. In another particular embodiment, the attachment means are preferably located at the upper part of the introducer, wherein the upper part is referred to as the upside of the device when held in a position suitable for use. Most preferably, said attachment means and said connecting means are located substantially opposite with respect to each other, thereby allowing them to easily cooperate with each other for allowing the partial release of the hollow tube and tube holder.

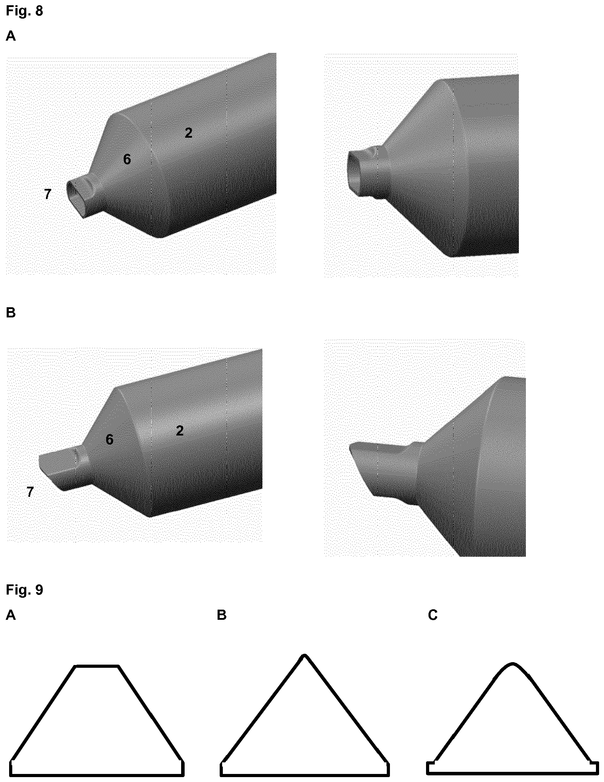

In order to drive the implant out of the hollow tube, a piston (8) is provided, with a plunger (9) and a pushrod (10). One of the obstacles to overcome in implant introducers is the risk of the implant getting stuck between the plunger and the walls of the hollow tube, thereby increasing the risk of damaging the implant. Therefore, the plunger is preferably designed such as to reduce the risk of the implant to become stuck in the device, or not being completely pushed out of the device (see figure 9A-C). In a particular embodiment, said plunger is thus adapted to conform to the inner contours of the tapered flange (6) of said hollow tube. Hence, the plunger is preferably conical with a blunt tip (Fig. 9B, C). However, said plunger may also be semi-conical, wherein the tip is flat over a diameter corresponding to the diameter of the injection opening (Fig. 9A). Said latter embodiment allows the implant to squeeze through at once. Also the widened area puts less stress on a concentrated point decreasing damage to the implant.

Another problem, which may occur, is that the implant is not fully pushed out of the device, but remains partially stuck in the injection tip. Due to the fact that the tip is completely inserted into the body cavity, the surgeon cannot see that the implant remains stuck. Therefore, in a particular embodiment, the plunger may be made from 2 parts, a body part, which conforms to the shape of the hollow tube, and a top part, which conforms to the shape of the injection tip, which parts cooperate to push the implant out of the device. When the plunger reaches the end of the hollow tube, the top part can be separately driven, pushed into the injection tip and as such pushes the final part of the implant out of the injection tip.

The plunger may be made from a single type of material or may be made from a combination of multiple materials. For example, the core of the plunger may be made from stainless steel, which is surrounded by a coating of for example PEEK. This may be advantageous, in particular in the event that the hollow tube is also made from stainless steel, thereby avoiding metal/metal friction. Also, the whole plunger can be made from one non-metallic material.

In another particular embodiment, said implant introducer further comprises a driving mechanism attached to said piston, for driving the displacement of said plunger within said hollow tube. The piston can for example be driven by manual, mechanical or pneumatic means. However, since damage to the implant can occur when forces applied to the implant are too high, the piston is preferably driven by means allowing the applied force to be controlled. For example the piston can be driven using a driving mechanism as generally applied in silicone gun systems, such as for example depicted in figure 2.

Using such a driving mechanism, at rest, the pad (19) is pushed against a first scum (15a) by means of a first spring (16a). The second spring (16b) pushes the slide (17) into a tilted position, such that the pushrod (10) is blocked. The orientation of the slide (17) ensures that a forward movement is possible, but a backward movement is hindered until the plate is again untilted. By actuating the pinching arm, the pivot point will follow its movement, and pull the pad down, such that it will clamps around the pushrod (10) and pushes it forward. After releasing the pinching arm, the first spring (16a) will have the tendency to push the pad back against the second scum (16b) which also serves as a stop. This ensures that the pinching arm will return to its original position and is again ready for the next move.

The first spring (16a) thus in fact serves two purposes, i.e. allowing the pad to be pushed backward after actuation, and for providing a certain amount of resistance for the user to get a good feeling of the device. Hence the first spring should have sufficient power to push the pad backward, while at the same time not being to strong for the surgeon to use. Furthermore, the first string is preferably linear, such that an equal force can be applied at all times. The second spring (16b), should not be too strong, since it will only be used to push the slide (17) into a tilted position to block the pushrod (10), this is usually done by pushing the slide with a thumb. The housing for the driving mechanism is preferably kept as simple as possible such that it can easily be sterilized.

Although the implant introducer and the different parts thereof may be provided in varying shapes and sizes, the different parts of the implant introducer are preferably provided in one or more of the following dimensions:
- the inner diameter of said hollow tube is about and between 60-100 mm, preferably about and between 70 - 90 mm, more preferably about 80 mm; and/or
- the diameter of said injection opening is about and between 10-50 mm, preferably about and between 20-40 mm, more preferably about 25 mm; and/or
- said injection opening ends into an injection tip being about and between 1-20 mm in length, preferably about 15 mm in length; and/or
- the injection tip can be of various shapes: concave, convex, round, oval, or shaped as shown in fig 6a both horizontally as vertically.
- the hollow tube is between and about 10-50 cm in length, preferably about and between 10-40 cm, more preferably about 25 cm; and/or
- the angle of the tapered flange, represented by α, is about 30-60°, more preferably about 45; and/or
- the width of the push rod is between and about 5-20 mm, preferably about 10mm;
- the cross section of the hollow tube is stadium-shaped, cylindrical, oval, concave or convex in shape; preferably stadium-shaped or cylindrical; and/or
- the cross section of the injection tip is stadium-shaped.

With reference to these dimension it is noted that the injection tip is preferably kept as short as possible, since this is the smallest diameter through which the implant needs to be pushed. The longer the tip, the harder it is to inject the implant. However, a too short length makes it more difficult during surgery to keep the device into place. Furthermore, since the implant is preferably inserted behind the pectoral muscles the injection tip is preferably about 15mm in length, since this is about the distance between the incision area and the cavity behind the beast muscles. In addition, the incision area is usually kept open using a retractor giving a certain shape to the incision. Hence, the injection tip is preferably of such size and shape to easily fit in said opening. The inventors have found that an injection tip being stadium-shaped is most suitable for this type of devices (see fig. 8A and 8B).

In the context of this invention a 'stadium-shape' is a 2-dimensional geometric shape constructed of a rectangle with semicircles at a pair of opposite sites, sometimes also referred to as discorectangle (see fig. 6A).

The rectangular sides of the injection tip may be different of height/length, such as the shape of a needle, but without sharp edges, i.e. herein referred to as elongated sides (see fig. 8B). When holding the device under the right angle, the longer height of the injection tip on one side then makes it easier to keep the device in place, while the shorter height of the other side results in the implant to be pushed out more easily. Furthermore, such elongated sides may also aid in orienting the injection device correctly during surgery.

The elongated side also acts as an internal retractor, this means that there is no need to hold a separate retractor in the incision during the squeezing of the implant. The most commonly used retractor is an L shaped hook of which one end is put inside the incision and pulled from the other side in order to open the incision. There are 2 problems with using such a retractor:
1. It has to be done by the assistant as the surgeon has his hands full while holding the injection device. This is the ONLY time an assistant is needed during this procedure.
2. There is a risk (1 in 5 patients) that while opening the incision using the retractor, the skin tears apart, resulting in a more difficult suture and a more visible scar.
The advantage of using an ejection tip with elongated sides is to avoid both these problems and it can be cost saving as there is no more assistant needed.

Also the angle of the tapered flange is very relevant in the context of the present invention. Whenever the hollow tube would not have a tapered flange or would have an angle α, which is too small, the introduction of the implant would be more difficult, since the device could be held too close to the body area, thereby hindering the surgeon. By providing an ideal angle α, as depicted in figure 5, the surgeon would automatically hold the device in the correct position, without having to worry about it. Furthermore, the correct angle also puts the least amount of stress on the implant during injection, thereby reducing the risk of damaging the implant.

The cross section of the hollow tube may be of any shape suitable for accommodating an implant. For example, the cross section of the hollow tube may be cylindrical, oval, concave, convex or any combination thereof; preferably cylindrical. Alternatively, the cross section of the hollow tube may comprise a combination of bend and straight surfaces, such as for example 2 opposite straight surface in combination with 2 opposite bend surfaces (see figure 6a); or one bend surface in combination with one straight surface (see figure 6b). While the transition of the hollow tube into the tapered flange and finally into the injection tip, may be sharp (see figure 7A), these transitions are preferably smooth such as to reduce the risk of damaging the implant (see figure 7B).

The materials used for the implant introducer, and in particular for the hollow tube, must be chosen such that they are reliable and safe during an operation. It is also very important that the chosen materials are easy to sterilize and can withstand the high temperatures of an autoclave. Furthermore, the materials must comply with certain mechanical and physical properties, such as being resistant to the forces applied during use of the device. Evidently, these considerations apply, regardless of whether the device is provided as a completely disposable device or as reusable device. Hence in a particular embodiment, the implant introducer of the present invention is preferably made from a sterilizable biocompatible materials such as for example selected from the non-limiting list comprising stainless steel, glass, acrylics, thermoplastics, carbon fiber, poly-urethane, poly-propylene or poly-ethylene.

When using the implant introducer of the present invention, there are several advantages in comparison to manual introduction of an implant. These advantages become apparent when comparing both methods.
In general the surgical procedure using an implant introducer comprises the following steps:
1) removal of the implant introducer from the sterile packaging
2) removal of the implants from the sterile packaging and applying a suitable, biocompatible, for internal use accepted, lubricating agent such as for example Instillagel
3) opening the implant introducer, and applying further lubricating agent in the hollow tube, while simultaneous sealing off the injection opening (7), for example by pressing a thumb against or into the injection opening
4) inserting the implant into the introducer, and closing the implant introducer again
5) removing the sealing of the injection opening and simultaneously introducing the implant introducer into the incision area
6) keeping the device under a suitable angle for introduction
7) driving the piston for ejecting the implant out of the device and into the incision
8) removing the device from the incision area.

| | Manual introduction | Implant Introducer |
|---|---|---|
| Incision | ± 7 cm | ± 3 cm |
| Scar | ± 7 cm | ± 3 cm |
| Operating Time | ± 1 u | ± 25 min |

The comparison between the implant introducer method, and the manual introduction, reveals that the implant introducer method provides a smaller, thinner and less visible scar and a shorter rehabilitation period. The operation time is considerably shorter, hence the patient is exposed to general anaesthesia for a shorter period of time, and the wound is also open for a shorter period of time, thereby reducing bacterial infiltration and thus reducing the risk of infections. With this method, the surgeon can perform twice as many operations in the same amount of time compared to the traditional manual method.

Furthermore, the advantages of the new implant introducer over the prior art known introducers have been discussed herein above and in particular reside in a shorter operation time and an increased sterility during operation, due to the specific characteristics of the device.

## Claims

1. A breast implant introducer (1) comprising:
- a hollow tube (2) comprising a first (3) and second longitudinal end (4), said first longitudinal end having a receiving opening (5) for receiving said implant; said second longitudinal end having a tapered flange (6) and an injection opening (7) for injecting said implant;
- a piston (8) comprising a plunger (9) and a pushrod (10); said plunger being adapted to conform to the inner contours of the tapered flange (6) of said hollow tube;
**characterized in that** said breast implant introducer further comprises a tube holder (11) having an opening (12) for receiving the pushrod of said piston; and releasing means comprising attachment means (13) and connecting means (14), which allow the hollow tube to be fully or partially disconnected from said tube holder; and **in that** the angle of the tapered flange, represented by α, is between 30° and 60°.

2. The breast implant introducer according to claim 1, further comprising a driving mechanism attached to said piston, for driving the displacement of said plunger within said hollow tube.

3. The breast implant introducer according to anyone of claims 1 to 2, wherein said attachment means (13) comprise a first attachment member (13a) located at the first longitudinal end of said hollow tube, which is adapted to cooperate with a second attachment member (13b) located at the tube holder, and which allow the hollow tube to be fully disconnected from the tube holder.

4. The breast implant introducer according to claim 3, wherein said first attachment member (13a) consists of an elevated ring (21) at the outer surface of the hollow tube near the opening for receiving the pushrod (12), and said second attachment member (13b), which is located at the inner surface of the tube holder, consists of a spring-actuated pivoting member (22) having a tapered flange (23) and a groove (24), said groove adapted to confirm to the size and shape of the elevated ring (21).

5. The breast implant introducer according to anyone of claims 1 to 2, wherein said attachment means (13), comprise a first attachment member (13a) located at the first longitudinal end of said hollow tube, which is adapted to cooperate with a second attachment member (13b) located at the tube holder; and wherein said connecting means (14), comprise a first connecting member (14a) located at the first longitudinal end of said hollow tube, which is adapted to cooperate with a second connecting member (14b) located at the tube holder; wherein said connecting means allow the hollow tube to be partially disconnected from said tube holder, upon release of said attachment means.

6. The breast implant introducer according to claim 5, wherein said first attachment member (13a) and said second attachment member (13b) comprise a tab and an aperture adapted for receiving said tab.

7. The breast implant introducer according to claim 5, wherein said first connecting member (14a) and said second connecting member (14b) comprise a hook and a pin adapted for receiving said hook, or a pin and an aperture for receiving said pin.

8. The breast implant introducer according to anyone of claims 1 to 7, wherein the inner diameter of said hollow tube is about and between 60-100 mm.

9. The breast implant introducer according to anyone of claims 1 to 8, wherein the diameter of said injection opening is about and between 10-50 mm.

10. The breast implant introducer according to anyone of claims 1 to 9, wherein said injection opening ends into an injection tip being about and between 1-30 mm in length.

11. The breast implant introducer according to anyone of claims 1 to 10, wherein the hollow tube is between and about 10-50 cm in length.

12. The breast implant introducer according to anyone of claims 1 to 11; wherein the angle of the tapered flange, represented by α, is about 45°.

13. The breast implant introducer according to anyone of claims 1 to 12, wherein said introducer is made from a sterilizable biocompatible material such as for example selected from the list comprising stainless steel, poly-urethane, poly-propylene, poly-ethylene, glass or acrylics.

14. The breast implant introducer according to anyone of claims 1 to 13, wherein the cross section of the hollow tube is stadium-shaped, cylindrical, oval, concave or convex in shape; preferably stadium-shaped or cylindrical.

15. The breast implant introducer according to anyone of claims 1 to 14, wherein the cross section of the injection tip is stadium-shaped.

## Patentansprüche

1. Brust-Implantat Zuführvorrichtung (1), umfassend:
- ein Hohlrohr (2), welches ein erstes (3) und ein zweites longitudinales Ende (4) umfasst, wobei das erste longitudinale Ende eine Aufnahmeöffnung (5) zur Aufnahme des Implantats aufweist; wobei das zweite longitudinale Ende einen konischen Flansch (6) und eine Injektionsöffnung (7) zum Injizieren des Implantats aufweist;
- einen Kolben (8), welcher einen Druckkolben (9) und eine Druckstange (10) umfasst, wobei der Druckkolben adaptiert ist, den inneren Konturen des konischen Flansches (6) des Hohlrohrs zu entsprechen;
**dadurch gekennzeichnet, dass** die Brust-Implantat Zuführvorrichtung weiter einen Rohrhalter mit einer Öffnung (12) zur Aufnahme der Druckstange des Kolbens umfasst; und Lösemittel umfassend Befestigungsmittel (13) und Verbindungsmittel (14) aufweist, welche dem Hohlrohr ermöglichen, vollständig oder teilweise von dem Rohrhalter getrennt zu werden; und dass der Winkel des konischen Flansches, der durch α, repräsentiert wird, zwischen 30° und 60° liegt.

2. Brust-Implantat Zuführvorrichtung nach Anspruch 1, welche weiter einen Antriebsmechanismus umfasst, der an dem Kolben angebracht ist, um die Verschiebung des Druckkolbens innerhalb des Hohlrohrs anzutreiben.

3. Brust-Implantat Zuführvorrichtung nach einem der Ansprüche 1 bis 2, wobei die Befestigungsmittel (13) ein erstes Befestigungselement (13a) umfassen, welches sich an dem ersten longitudinalen Ende des Hohlrohrs befindet, und das adaptiert ist, mit einem zweiten Befestigungselement (13b), welches sich an dem Rohrhalter befindet, zusammenzuarbeiten, und die dem Hohlrohr erlauben, vollständig von dem Rohrhalter getrennt zu werden.

4. Brust-Implantat Zuführvorrichtung nach Anspruch 3, wobei das erste Befestigungselement (13a) aus einem erhöhten Ring (21) an der äußeren Oberfläche des Hohlrohrs nahe der Öffnung zur Aufnahme der Druckstange (12) besteht, und das zweite Befestigungselement (13b), welches sich an der inneren Oberfläche des Rohrhalters befindet, aus einem durch Federkraft betätigten Schwenkelement (22) mit einem konischen Flansch (23) und einer Nut (24) besteht, wobei die Nut adaptiert ist, der Größe und Form des erhöhten Rings (21) zu entsprechen.

5. Brust-Implantat Zuführvorrichtung nach einem der Ansprüche 1 bis 2, wobei die Befestigungsmittel (13) ein erstes Befestigungselement (13a) umfassen, welches sich an dem ersten longitudinalen Ende des Hohlrohrs befindet, welches adaptiert ist, mit einem zweiten Befestigungselement (13b) zusammenzuarbeiten, welches sich am Rohrhalter befindet; und wobei die Verbindungsmittel (14) ein erstes Verbindungselement (14a) umfassen, welches sich an dem ersten longitudinalen Ende des Hohlrohrs befindet, welches adaptiert ist, mit einem zweiten Verbindungselement (14b) zusammenzuarbeiten, welches sich am Rohrhalter befindet; wobei die Befestigungsmittel dem Hohlrohr erlauben, bei Freigabe der Befestigungsmittel teilweise von dem Rohrhalter getrennt zu werden.

6. Brust-Implantat Zuführvorrichtung nach Anspruch 5, wobei das erste Befestigungselement (13a) und das zweite Befestigungselement (13b) einen Nase und eine Blende, welche adaptiert ist, die Nase aufzunehmen, umfassen.

7. Brust-Implantat Zuführvorrichtung nach Anspruch 5, wobei das erste Verbindungselement (14a) und das zweite Verbindungselement (14b) einen Haken und einen Stift, welcher adaptiert ist, den Haken aufzunehmen, oder einen Stift und eine Blende zur Aufnahme des Stifts umfassen.

8. Brust-Implantat Zuführvorrichtung nach einem der Ansprüche 1 bis 7, wobei der innere Durchmesser des Hohlrohrs um und zwischen 60-100 mm liegt.

9. Brust-Implantat Zuführvorrichtung nach einem der Ansprüche 1 bis 8, wobei der Durchmesser der Injektionsöffnung um und zwischen 10-50 mm liegt.

10. Brust-Implantat Zuführvorrichtung nach einem der Ansprüche 1 bis 9, wobei die Injektionsöffnung in einer Injektionsspitze endet, deren Länge um und zwischen 1-30 mm liegt.

11. Brust-Implantat Zuführvorrichtung nach einem der Ansprüche 1 bis 10, wobei das Hohlrohr eine Länge von zwischen und um 10-50 mm hat.

12. Brust-Implantat Zuführvorrichtung nach einem der Ansprüche 1 bis 11, wobei der Winkel des konischen Flansches, welcher durch α repräsentiert wird, um 45° liegt.

13. Brust-Implantat Zuführvorrichtung nach einem der Ansprüche 1 bis 12, wobei die Zuführvorrichtung aus sterilisierbarem, biokompatiblem Material wie zum Beispiel ausgewählt aus der Gruppe, umfassend Edelstahl, Polyurethan, Polypropylen, Polyethylen, Glas oder Acryl hergestellt wird.

14. Brust-Implantat Zuführvorrichtung nach einem der Ansprüche 1 bis 13, wobei der Querschnitt des Hohlrohrs eine stufenförmig zylindrische, zylindrische, ovale, konkave oder konvexe Form hat, vorzugsweise stufenförmig zylindrisch oder zylindrisch.

15. Brust-Implantat Zuführvorrichtung nach einem der Ansprüche 1 bis 14, wobei der Querschnitt der Injektionsspitze stufenförmig zylindrisch ist.

## Revendications

1. Introducteur d'implants mammaires (1) comprenant :
- un tube creux (2) présentant une première extrémité longitudinale (3) et une seconde (4), ladite première extrémité longitudinale possédant une ouverture réceptrice (5) destinée à accueillir ledit implant, ladite seconde extrémité longitudinale présentant un collet conique (6) et une ouverture d'injection (7) pour l'injection dudit implant ;
- un piston (8) comprenant un poussoir (9) et une tige de poussée (10), ledit poussoir étant façonné pour épouser parfaitement les contours internes du collet conique (6) dudit tube creux ;
ledit introducteur d'implants mammaires se **caractérise par le fait qu'**il comprend également un support de tube possédant une ouverture (12) pour le passage de la tige de poussée dudit piston ; et des moyens de désengagement comprenant des moyens d'accroche (13) et des moyens de connexion (14), qui permettent au tube creux d'être totalement ou partiellement déconnecté dudit support de tube ; et il se **caractérise par le fait que** l'angle du collet conique, représenté par le signe α,, se situe entre 30° et 60°.

2. Introducteur d'implants mammaires selon la revendication 1, comprenant en outre un mécanisme d'entraînement raccordé audit piston, destiné à entraîner le déplacement dudit poussoir à l'intérieur dudit tube creux.

3. Introducteur d'implants mammaires selon l'une quelconque des revendications 1 à 2, dans lequel lesdits moyens d'accroche (13) comprennent un premier élément d'accroche (13a) situé au niveau de la première extrémité longitudinale dudit tube creux et conçu pour fonctionner solidairement avec un second élément d'accroche (13b) situé au niveau du support de tube, qui permettent au tube creux d'être totalement déconnecté du support de tube.

4. Introducteur d'implants mammaires selon la revendication 3, dans lequel ledit premier élément d'accroche (13a) correspond à une bague surélevée (21) à la surface externe du tube creux près de l'ouverture de passage de la tige de poussée (12), et dans lequel ledit second élément d'accroche (13b), qui est situé à la surface interne du support de tube, correspond à un élément pivotant actionné par ressort (22), présentant un rebord conique (23) et une gorge (24), ladite gorge étant façonnée pour être parfaitement adaptée à la taille et à la forme de la bague surélevée (21).

5. Introducteur d'implants mammaires selon l'une quelconque des revendications 1 à 2, dans lequel lesdits moyens d'accroche (13) comprennent un premier élément d'accroche (13a) situé au niveau de la première extrémité longitudinale dudit tube creux et conçu pour fonctionner solidairement avec un second élément d'accroche (13b) situé au niveau du support de tube ; et dans lequel lesdits moyens de connexion (14) comprennent un premier élément de connexion (14a) situé au niveau de la première extrémité longitudinale dudit tube creux et conçu pour fonctionner solidairement avec un second élément de connexion (14b) situé au niveau du support de tube ; dans lequel lesdits moyens de connexion permettent au tube creux d'être partiellement déconnecté dudit support de tube après désengagement desdits moyens d'accroche.

6. Introducteur d'implants mammaires selon la revendication 5, dans lequel ledit premier élément d'accroche (13a) et ledit second élément d'accroche (13b) comprennent une languette et un orifice façonné pour accueillir ladite languette.

7. Introducteur d'implants mammaires selon la revendication 5, dans lequel ledit premier élément de connexion (14a) et ledit second élément de connexion (14b) comprennent un crochet et un picot façonné pour accueillir ledit crochet, ou un picot et un orifice façonné pour accueillir ledit picot.

8. Introducteur d'implants mammaires selon l'une quelconque des revendications 1 à 7, dans lequel le diamètre interne dudit tube creux est approximatif et compris entre 60 et 100 mm.

9. Introducteur d'implants mammaires selon l'une quelconque des revendications 1 à 8, dans lequel le diamètre de ladite ouverture d'injection est approximatif et compris entre 10 et 50 mm.

10. Introducteur d'implants mammaires selon l'une quelconque des revendications 1 à 9, dans lequel ladite ouverture d'injection se termine par un embout d'injection dont la longueur est approximative et comprise entre 1 et 30 mm.

11. Introducteur d'implants mammaires selon l'une quelconque des revendications 1 à 10, dans lequel la longueur du tube creux est approximative et comprise entre 10 et 50 cm.

12. Introducteur d'implants mammaires selon l'une quelconque des revendications 1 à 11, dans lequel l'angle du collet conique, représenté par le signe α, est d'environ 45°.

13. Introducteur d'implants mammaires selon l'une quelconque des revendications 1 à 12, dans lequel ledit introducteur est fabriqué dans un matériau biocompatible stérilisable, choisi par exemple dans la liste comprenant l'acier inoxydable, le polyuréthane, le polypropylène, le polyéthylène, le verre et l'acrylique.

14. Introducteur d'implants mammaires selon l'une quelconque des revendications 1 à 13, dans lequel la coupe transversale du tube creux est de forme rectangle avec angles arrondis, cylindrique, ovale, concave ou convexe, avec une préférence pour la forme rectangle avec angles arrondis ou la forme cylindrique.

15. Introducteur d'implants mammaires selon l'une quelconque des revendications 1 à 14, dans lequel la coupe transversale de l'embout d'injection est en forme de rectangle avec angles arrondis.
